# EUROPEAN PATENT SPECIFICATION

(11) **EP 2 896 358 B1**
(45) Date of publication and mention of the grant of the patent: **04.01.2017**
(21) Application number: 15150971.8
(22) Date of filing: 13.01.2015
(51) Int. Cl.: A61B 5/00, A61B 5/02, A61B 5/021, A61B 5/022, A61B 5/0235

(54) **Apparatus for evaluating vascular endothelial function**
Vorrichtung zum Beurteilen der vaskulären Endothelfunktion
Appareil d'évaluation de la fonction endothéliale vasculaire

(30) Priority: 21.01.2014 JP 2014008465
(43) Date of publication of application: 22.07.2015
(73) Proprietor: Nihon Kohden Corporation, Shinjuku-ku Tokyo (JP)
(72) Inventor: Ono, Yoshinobu, Tokyo (JP); Takayanagi, Tsuneo, Tokyo (JP)
(74) Representative: Brevalex

(56) References cited:
- EP-A1- 2 294 976
- EP-A1- 2 606 818
- WO-A1-2012/149209
- CN-U- 202 909 061
- JP-A- 2004 129 979

## Description

### BACKGROUND

The present invention relates to an apparatus for evaluating a vascular endothelial function in which avascularization is performed by using a cuff, a pulse wave is detected before and after the avascularization is detected, and a vascular endothelial function is evaluate based on the detected pulse waves.

The assignees of the present application have filed patent applications on apparatuses for evaluating a vascular endothelial function which, similarly with the above, use cuffs, and in which accurate evaluation similar to that obtained in a measurement using an ultrasonic echo system is enabled without using an ultrasonic echo system or the like (see JP-A-2009-273870 and JP-A-2011-056200).

In the apparatuses disclosed in the literatures, a vascular endothelial function is evaluated by comparing pulse waves before and after avascularization in place of a measurement in which the change of the vessel diameter before and after avascularization is measured by using an ultrasonic echo system.

According to the apparatuses for evaluating a vascular endothelial function disclosed in JP-A-2009-273870 and JP-A-2011-056200, accurate evaluation of a vascular endothelial function is enabled, but there may be a risk that the amplitude of a pulse wave is varied by influence of respiration and autonomic nerves in addition to an expansion of the vessel diameter caused by avascularization.

The invention has been conducted in view of the present status of an apparatus for evaluating a vascular endothelial function which uses cuffs. It is an object of the invention to provide an apparatus for evaluating a vascular endothelial in which accurate evaluation of a vascular endothelial function is enabled without being influenced by respiration and autonomic nerves.

### SUMMARY

In one of its aspects, the apparatus for evaluating a vascular endothelial function of the invention is characterized in that the apparatus includes: a first cuff which is to be wrapped around one of right and left upper or lower limbs of a subject; a second cuff which is to be wrapped around one of the right and left upper or lower limbs, the one limb being to be wrapped by the first cuff; a third cuff which is to be wrapped around another one of the right and left upper or lower limbs, the other one limb being opposite to the one limb around which the first cuff and the second cuff are to be wrapped; a cuff pressure controller which controls pressures that are applied to the cuffs, respectively; a cuff pressure detector which, from an output of a pressure sensor connected to the second cuff, and an output of a pressure sensor connected to the third cuff, detects cuff pressures in the respective cuffs; a pulse wave detector which detects respective pulse waves from outputs of the pressure sensors; and an analyzer which analyzes the detected pulse waves to evaluate a vascular endothelial function, the cuff pressure controller controls the cuff pressure of the first cuff to avascularize the upper or lower limb around which the first cuff is wrapped, for a predetermined time period, the pulse wave detector detects a pulse wave corresponding to the second cuff, and a pulse wave corresponding to the third cuff, before and after the avascularization in a state where the cuff pressures of the second and third cuffs are controlled to respective predetermined pressure by the cuff pressure controller, and the analyzer corrects the pulse waves corresponding to the second cuff and detected before and after the avascularization, by the pulse waves corresponding to the third cuff and detected before and after the avascularization, and evaluates the endothelial function based on the corrected pulse waves corresponding to the second cuff.

In another of its aspects, the apparatus for evaluating a vascular endothelial function of the invention is characterized in that the apparatus includes: a second cuff which is to be wrapped around one of right and left upper or lower limbs of a subject; a third cuff which is to be wrapped around another one of the right and left upper or lower limbs, the other one limb being opposite to the one limb around which the second cuff is to be wrapped; a cuff pressure controller which controls pressures that are applied to the cuffs, respectively; a cuff pressure detector which, from an output of a pressure sensor connected to the second cuff, and an output of a pressure sensor connected to the third cuff, detects cuff pressures in the respective cuffs; a pulse wave detector which detects respective pulse waves from outputs of the pressure sensors; and an analyzer which analyzes the detected pulse waves to evaluate a vascular endothelial function, the cuff pressure controller controls the cuff pressure of the second cuff to avascularize the upper or lower limb around which the second cuff is wrapped, for a predetermined time period, the pulse wave detector detects a pulse wave corresponding to the second cuff, and a pulse wave corresponding to the third cuff, before and after the avascularization in a state where the cuff pressures of the second and third cuffs are controlled to respective predetermined pressures by the cuff pressure controller, and the analyzer corrects the pulse waves corresponding to the second cuff and detected before and after the avascularization, by the pulse waves corresponding to the third cuff and detected before and after the avascularization, and evaluates the vascular endothelial function based on the corrected pulse waves corresponding to the second cuff.

The apparatus for evaluating a vascular endothelial function of the invention may also be characterized in that the pulse waves handled in the analyzer are pulse wave indexes, and the pulse wave indexes are a parameter indicating a feature of a pulse wave.

The apparatus for evaluating a vascular endothelial function of the invention may be characterized in that the correction corrects a result of a comparison between the pulse wave indexes corresponding to the second cuff and detected before and after the avascularization.

The apparatus for evaluating a vascular endothelial function of the invention may be characterized in that the comparison result is ratios of the pulse wave indexes of the second cuff and the third cuff before and after the avascularization, and the correction corrects the ratio of the pulse wave indexes corresponding to the second cuff by using the ratio of the pulse wave indexes corresponding to the third cuff.

The apparatus for evaluating a vascular endothelial function of the invention may be characterized in that the comparison and the correction are performed by using values of the pulse wave indexes corresponding to the second cuff or the third cuff, the values being obtained at a required timing, and values of the pulse wave indexes corresponding to the third cuff or the second cuff, the values being obtained at the required timing.

The apparatus for evaluating a vascular endothelial function of the invention may be characterized in that the comparison and the correction are performed by using values of the pulse wave indexes corresponding to the third cuff, and values of the pulse wave indexes corresponding to the second cuff and equal to the values of the pulse wave indexes corresponding to the third cuff.

The apparatus for evaluating a vascular endothelial function of the invention may be characterized in that the pulse wave indexes are a maximum value of a pulse wave amplitude, or a mean value in the vicinity of the maximum value.

The apparatus for evaluating a vascular endothelial function of the invention may be characterized in that the analyzer further performs a process of obtaining a systolic blood pressure and a diastolic blood pressure, and dividing an amplitude of the detected pulse wave by a difference between the systolic blood pressure and the diastolic blood pressure, thereby obtaining a compliance of a blood vessel.

According to the apparatus for evaluating a vascular endothelial function of the invention, the pulse waves corresponding to the second cuff and detected before and after the avascularization are corrected by those corresponding to the third cuff and detected before and after the avascularization, and the vascular endothelial function is evaluated based on the corrected pulse waves corresponding to the second cuff. Therefore, the vascular endothelial function can be accurately evaluated.

Namely, the pulse waves corresponding to the second cuff are presumed to contain an influence caused by an expansion of the vessel diameter due to the avascularization, and influences of respiration and autonomic nerves, and by contrast those corresponding to the third cuff are presumed to contain influences of respiration and autonomic nerves. When the pulse waves corresponding to the second cuff and containing an influence caused by an expansion of the vessel diameter due to the avascularization, and influences of respiration and autonomic nerves are corrected by those corresponding to the third cuff and containing influences of respiration and autonomic nerves, therefore, it is possible to take out only information relating to the expansion of the vessel diameter due to the avascularization. Therefore, it is expected to accurately evaluate a vascular endothelial function without being influenced by respiration and autonomic nerves.

### BRIEF DESCRIPTION OF DRAWINGS

Fig. 1 is a block diagram of an apparatus for evaluating a vascular endothelial function of an embodiment of the invention.
Fig. 2 is a diagram illustrating a state where cuffs of the apparatus for evaluating a vascular endothelial function of the embodiment of the invention are attached to the body.
Figs. 3A to 3C are views showing in time series a first example of avascularization and pulse wave measurement in operation of the apparatus for evaluating a vascular endothelial function of the embodiment of the invention.
Fig. 4 is a flowchart illustrating the operation of avascularization and pulse wave measurement in operation of the apparatus for evaluating a vascular endothelial function of the embodiment of the invention.
Figs. 5A to 5C are views showing an example of a statistical process performed by the apparatuses for evaluating a vascular endothelial function of the embodiment of the invention.
Figs. 6A to 6C are views showing in time series a second example of avascularization and pulse wave measurement in operation of the apparatus for evaluating a vascular endothelial function of the embodiment of the invention.
Figs. 7A to 7C are views showing in time series a third example of avascularization and pulse wave measurement in operation of the apparatus for evaluating a vascular endothelial function of the embodiment of the invention.
Figs. 8A to 8D are views showing in time series a fourth example of avascularization and pulse wave measurement in operation of the apparatus for evaluating a vascular endothelial function of the embodiment of the invention.

### DETAILED DESCRIPTION OF EMBODIMENTS

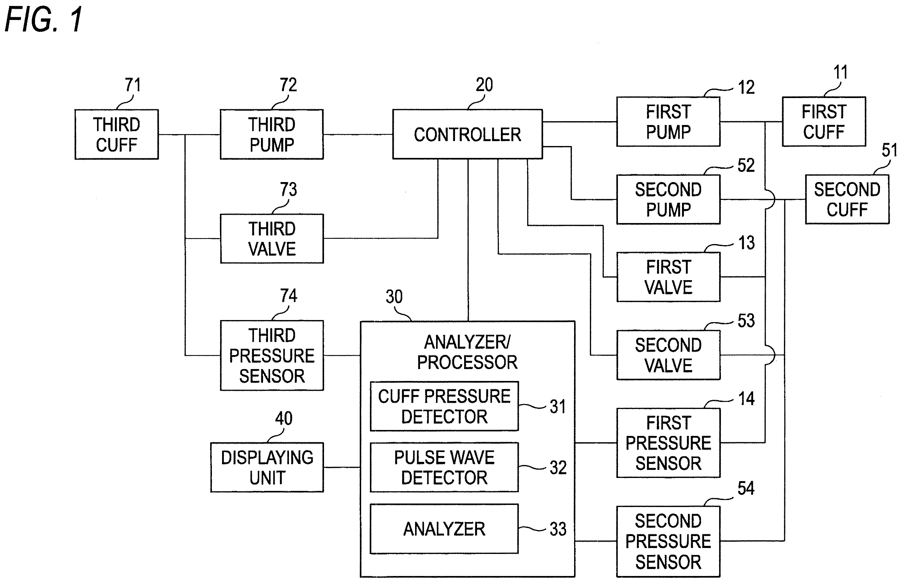

Fig. 1 is a block diagram of an apparatus for evaluating a vascular endothelial function of an embodiment of the invention. The apparatus includes a first cuff 11, a second cuff 51, and a third cuff 71. A first pump 12, a first valve 13, and a first pressure sensor 14 are connected to the first cuff 11. A second pump 52, a second valve 53, and a second pressure sensor 54 are connected to the second cuff 51. A third pump 72, a third valve 73, and a third pressure sensor 74 are connected to the third cuff 71. The apparatus further includes a controller 20, an analyzer/processor 30, and a displaying unit 40. The analyzer/processor 30 is connected to the controller 20, and the displaying unit 40 is connected to the analyzer/processor 30. The first pump 12, the second pump 52, the third pump 72, the first valve 13, the second valve 53, and the third valve 73 are connected to the controller 20, and the first pressure sensor 14, the second pressure sensor 54, and the third pressure sensor 74 are connected to the analyzer/processor 30.

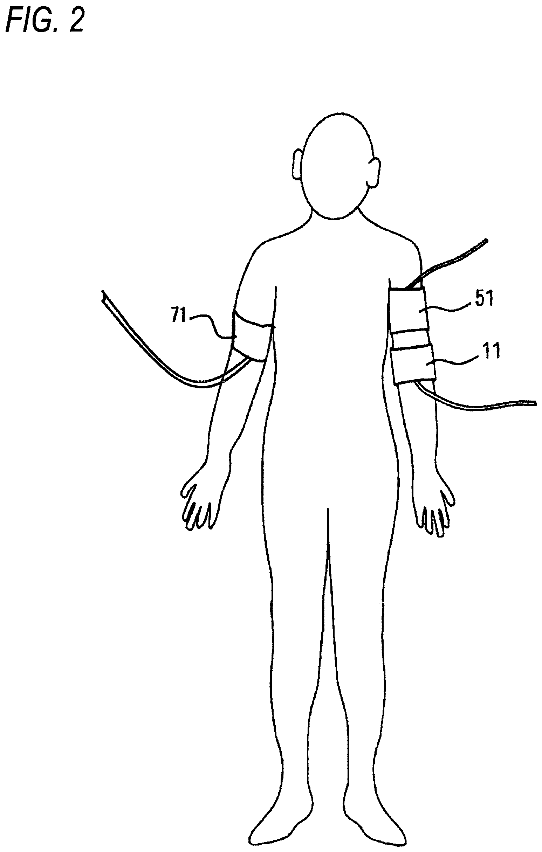

The first cuff 11 is to be wrapped around a first portion such as an upper or lower limb of the body of the subject, and used for applying a pressure for avascularization on the portion around which the cuff is wrapped. The second cuff 51 is to be wrapped around a second portion such as the upper or lower limb of the body of the subject on the side where the first cuff 11 is wrapped, and used for applying a pressure for detecting a pulse wave of the portion around which the cuff is wrapped. In a measurement, as shown in Fig. 2, for example, the first cuff 11 and the second cuff 51 are wrapped around an arm portion of the arm, and the second cuff 51 is preferably wrapped around a portion which is on the upstream side (the side which is closer to the heart) of the first cuff 11. The third cuff 71 is to be wrapped around a portion in the upper and lower limbs of the body, on the side (left or right side) which is different from the side (right or left side) around which the first cuff 11 and the second cuff 51 are wrapped, and used for applying a pressure for detecting a pulse wave of the portion around which the cuff is wrapped. In a measurement, in the case where the first cuff 11 and the second cuff 51 are wrapped around the left arm of the body as shown in Fig. 2, for example, the third cuff 71 is wrapped around the side of the right arm (side of the upper and lower limbs which is not avascularized).

The first pump 12 feeds the air into the first cuff 11 under the control of the controller 20. The first valve 13 switches non-discharging/discharging of the air in the first cuff 11 under the control of the controller 20. The second pump 52 feeds the air into the second cuff 51 under the control of the controller 20. The second valve 53 switches non-discharging/discharging of the air in the second cuff 51 under the control of the controller 20. The third pump 72 feeds the air into the third cuff 71 under the control of the controller 20. The third valve 73 switches non-discharging/discharging of the air in the third cuff 71 under the control of the controller 20. The controller 20 constitutes the cuff pressure controller which controls pressurization and depressurization of the first cuff 11, the second cuff 51, and the third cuff 71.

The first pressure sensor 14 outputs a signal corresponding to the pressure in the first cuff 11, the second pressure sensor 54 outputs a signal corresponding to the pressure in the second cuff 51, and the third pressure sensor 74 outputs a signal corresponding to the pressure in the third cuff 71. The analyzer/processor 30 controls the whole apparatus, is configured by, for example, a computer, and includes a cuff pressure detector 31, a pulse wave detector 32, and an analyzer 33. In the embodiment, for the sake of convenience, the controller 20 which controls the first cuff 11, the second cuff 51, and the third cuff 71, and the analyzer/processor 30 which performs analysis and processing are commonly used for the cuffs. Alternatively, they may be disposed for each of the cuffs.

The cuff pressure detector 31 detects the respective cuff pressures of the first, second, and third cuffs 11, 51, 71 from outputs of the first, second, and third pressure sensors 14, 54, 74. The pulse wave detector 32 detects pulse waves from the outputs of the second pressure sensor 54 and the third pressure sensor 74. The analyzer 33 calculates a systolic blood pressure and a diastolic blood pressure based on the detected pulse waves, and, as described later, analyzes pulse waves before and after the avascularization to evaluate a vascular endothelial function. The displaying unit 40 displays a result of the evaluation of the vascular endothelial function, the blood pressure value, and the like.

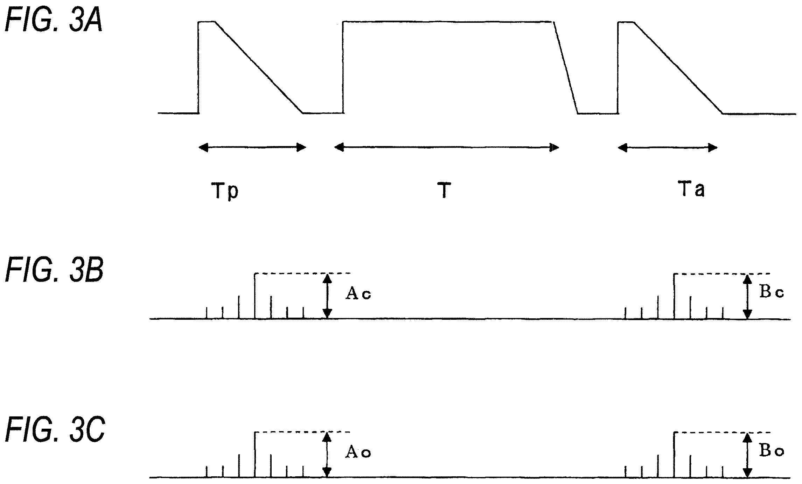

The controller 20 drives the first, second, and third pumps, and the first, second, and third valves to control the cuff pressures of the first, second, and third cuffs to a predetermined value. For example, the cuff pressures of the first cuff 11, the second cuff 51, and the third cuff 71 are changed as shown in Fig. 3A. Namely, avascularization is performed by using the first cuff 11 during a pressurization (avascularization) period T, and the pulse wave is measured before and after the pressurization period T, i.e., during measurement periods Tp and Ta by using the second cuff 51 and the third cuff 71. For example, the pressurization period T may be set to about five minutes, and the measurement periods Tp and Ta may be set to a time period which is required for usual blood pressure measurement. During the pressurization period T, the output of the first pressure sensor 14 is monitored, and avascularization is performed at a pressure which is a sum of the systolic blood pressure and a predetermined pressure (for example, 50 mmHg). In the measurement periods Tp and Ta, the output of the second pressure sensor 54 is monitored in the second cuff 51, that of the third pressure sensor 74 is monitored in the third cuff 71, the cuff pressures which are higher than the systolic blood pressure are gradually lowered, and pulse waves respectively corresponding to the cuffs are measured.

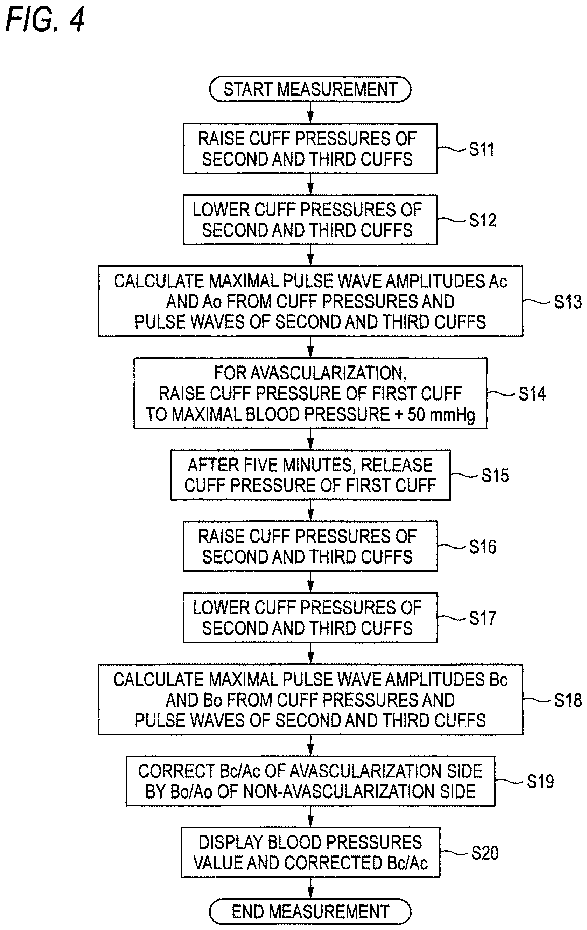

The apparatus for evaluating a vascular endothelial function can perform processing shown in, for example, the flowchart of Fig. 4. As described above, the first cuff 11 is wrapped around the left arm of the body of the subject as shown in, for example, Fig. 2, the second cuff 51 is wrapped around the left arm of the subject, and on the upstream side of the first cuff 11, the third cuff 71 is wrapped around the right arm of the body of the subject, and then the measurement is started. Under the control of the controller 20, the air is sent in a state where the second valve 53 is closed, from the second pump 52 to the second cuff 51, and, at the same time, from the third pump 72 to the third cuff 71 in a state where the third valve 73 is closed, to raise the cuff pressures (S11).

When the cuff pressures reaches a predetermined value, the air supplies from the second pump 52 and the third pump 72 are stopped, and the second valve 53 and the third valve 73 are controlled, whereby the cuff pressures are gradually lowered (S12).

During the process of lowering the cuff pressures, the cuff pressure detector 31 detects the cuff pressures from the outputs of the second pressure sensor 54 and the third pressure sensor 74, and the pulse wave detector 32 detects the pulse waves for each beat. Based on the cuff pressures and the pulse waves, the analyzer 33 obtains the values of the respective pulse wave indexes (S13). In the embodiment, the respective maximal pulse wave amplitudes are set as the pulse wave indexes. The maximal pulse wave amplitudes are obtained by statistically processing the pulse waves which are obtained in the measurement period Tp. The pulse wave in the measurement period Tp obtained by the second pressure sensor 54 is as shown in Fig. 3B, and the maximal pulse wave amplitude Ac is obtained as the value of the pulse wave index.

The blood pressure at the timing when the pulse wave shows the maximal amplitude in the measurement period Tp is the mean blood pressure. In the case where the mean blood pressure is previously known, therefore, pressurization to a pressure which is equal to or higher than the mean blood pressure is not necessary in order to obtain the maximal pulse wave, and the burden on the subject is reduced.

Similarly, the pulse wave in the measurement period Tp obtained by the third pressure sensor 74 is as shown in Fig. 3C, and the maximal pulse wave amplitude Ao is obtained in a similar manner as the maximal pulse wave amplitude Ac which is obtained from the pulse wave acquired by the second pressure sensor 54. In addition to the maximal pulse wave amplitude, a value in the vicinity of the maximal pulse wave amplitude (including a mean value), the velocity (first order differential of the pulse wave), the area, the acceleration (second order differential of the pulse wave), the time period until a peak, and the like may be used as the pulse wave index.

Next, the pressurization period T for avascularization when, in a state where the first valve 13 is closed under the control of the controller 20, the air is sent from the first pump 12 to the first cuff 11 to raise the cuff pressure, and avascularization is performed at a pressure which is a sum of the systolic blood pressure and the predetermined pressure (for example, 50 mmHg) is realized (S14). After five minutes, the first valve 13 is opened to release the cuff pressure, whereby the cuff pressure is lowered to a pressure which is equal to or lower than the systolic blood pressure (S15).

Thereafter, a state where the second valve 53 and the third valve 73 are closed is promptly set, the air is sent from the second pump 52 to the second cuff 51, and the air is sent from the third pump 72 to the third cuff 71, so that to the pressures of the cuffs are raised (S16). When the cuff pressures reach a predetermined value, the pressures of the second cuff 51 and the third cuff 71 are gradually lowered in a similar manner as described above (S17).

During the process of lowering the cuff pressures, the cuff pressures and the pulse waves for each beat are detected in a similar manner as step S13, and the analyzer 33 obtains the maximal pulse wave amplitudes Bc and Bo as the values of the pulse wave indexes from the cuff pressures and the pulse waves (S18).

Then, a result of comparison of the maximal pulse wave amplitudes Ac and Bc of the avascularization side is corrected by using that of comparison of the maximal pulse wave amplitudes Ao and Bo of the non-avascularization side (S19). Each of the comparisons can be performed by obtaining a result of a division of the maximal pulse wave amplitude Bc (Bo) by the maximal pulse wave amplitude Ac (Ao). Therefore, the comparison result of the avascularization side is (Bc/Ac), and that of the non-avascularization side is (Bo/Ao). The correction may be performed by dividing (Bc/Ac) of the comparison result of the avascularization side by (Bo/Ao) of the comparison result of the non-avascularization side, or by subtracting (Bo/Ao) of the comparison result of the non-avascularization side from (Bc/Ac) of the comparison result of the avascularization side.

The corrected maximal pulse wave amplitude ratio of the avascularization side is displayed together with the systolic blood pressure and the diastolic blood pressure, on the displaying unit 40 (S20). The vascular endothelial function is evaluated by the corrected maximal pulse wave amplitude values of the avascularization side which are displayed.

In the above, the comparison results are obtained first. Alternatively, the correction may be performed first. For example, the representative pulse wave amplitude Ac (Bc) of the avascularization side may be divided or subtracted by the representative pulse wave amplitude Ao (Bo) of the non-avascularization side to obtain the corrected pulse wave amplitude (for example, Ab) before avascularization and the corrected pulse wave amplitude (for example, Ba) after avascularization, and (Ba/Ab) may be obtained by comparison.

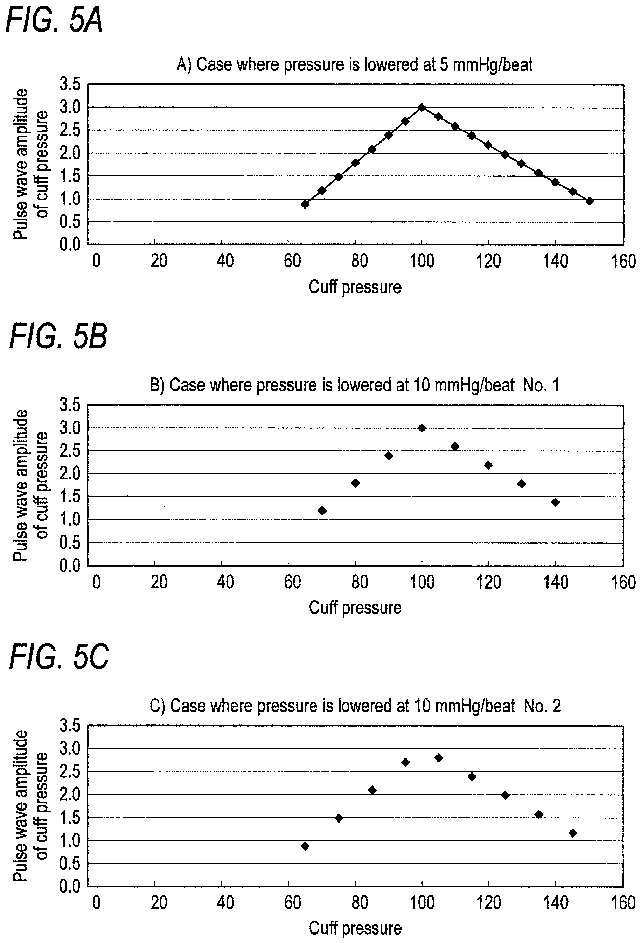

The relationship between the pulse wave amplitude and the cuff pressure is not constant. Figs. 5A to 5C show the relationship between the cuff pressure and the pulse wave amplitude. Fig. 5A shows the pulse wave amplitude in the case where the cuff pressure is lowered at the rate of 5 mmHg per beat, and Figs. 5B and 5C show the pulse wave amplitude in the case where the pressure reduction rate is increased. In the figures, the cuff pressure and pulse wave amplitude at the start of the pressure reduction are different.

In Figs. 5B and 5C, the maximal pulse wave amplitudes are 3 mmHg and 2.8 mmHg, respectively, and the difference of 0.2 mmHg is produced. The analyzer 33 performs statistical processing in which the mean value of a predetermined number (for example, three) of pulse wave amplitudes in the vicinity of the maximal pulse wave amplitude is calculated among obtained pulse wave amplitudes. In the example, when the mean value of pulse wave amplitudes at three points is calculated, the average is 2.67 mmHg in Fig. 5B, and 2.64 mmHg in Fig. 5C. The difference can be reduced, and a value which is preferred as the maximal pulse wave amplitude can be obtained. In the embodiment, it is assumed that the value of the maximal pulse wave amplitude includes a value in the vicinity of the maximal pulse wave amplitude (including a mean value).

In the above, during the measurement periods Tp and Ta, the maximum value of the pulse wave amplitude corresponding to the second cuff 51 (hereinafter, the amplitude is referred to as the pulse wave amplitude of the second cuff 51) is obtained, also the maximum value of the pulse wave amplitude corresponding to the third cuff 71 (hereinafter, the amplitude is referred to as the pulse wave amplitude of the third cuff 71) is similarly obtained, and the values are used in the correction. In the measurement periods Tp and Ta, however, the time when the pulse wave amplitude of the second cuff 51 is maximum is not always identical with that when the pulse wave amplitude of the third cuff 71 is maximum. Therefore, the correction may be performed by obtaining the pulse wave amplitude of the third cuff 71 at the time when the pulse wave amplitude of the second cuff 51 is maximum (the times when Ac and Bc are attained in Fig. 3B). Similarly, the correction may be performed by obtaining the pulse wave amplitude of the second cuff 51 at the time when the pulse wave amplitude of the third cuff 71 is maximum (the times when Ao and Bo are attained in Fig. 3C). When the timing when the pulse wave is acquired in the avascularization side is coincident with that in the non-avascularization side as described above, it is possible to realize a more correct correction.

Before avascularization, the pulse wave amplitudes of the second cuff 51 and the third cuff 71 may be obtained at the same timing, and, after avascularization, the timing when the pulse wave amplitude of the third cuff 71 of the non-avascularization side after avascularization is equal to that of the third cuff 71 before avascularization may be obtained. Then, the pulse wave amplitude of the second cuff 51 of the avascularization side at this timing may be obtained, and used as a value (hereinafter, referred to as the representative value) to be used in the comparison.

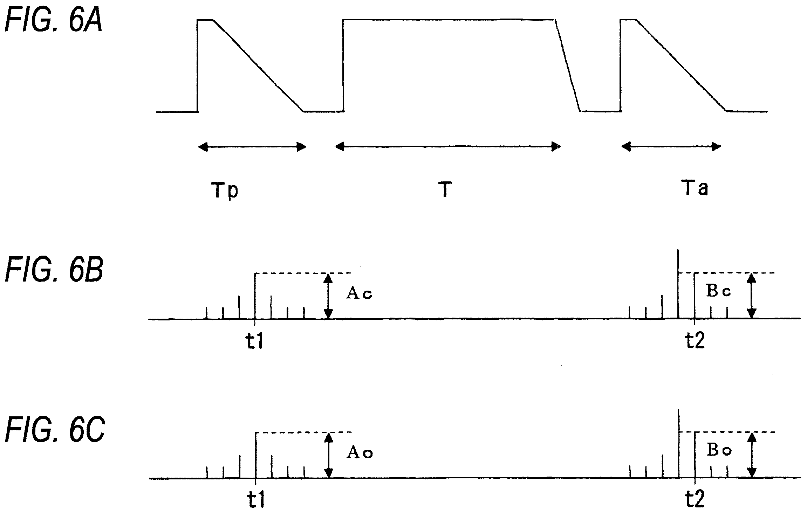

As shown in Figs. 6A to 6C, during the measurement period Tp before avascularization, for example, the maximal pulse wave amplitude Ac is obtained as the representative value from the pulse wave of the second cuff 51, and at the same time the pulse wave amplitude Ao is obtained as the representative value from the pulse wave of the third cuff 71 (time t1). During the measurement period Ta after avascularization, then, the time is obtained when the pulse wave of the third cuff 71 having the same amplitude as the pulse wave amplitude Ao which is obtained before avascularization is acquired. For example, it is assumed that the pulse wave amplitude Bo which is identical in amplitude with the pulse wave amplitude Ao that is obtained before avascularization is acquired at time t2, an then the obtained pulse wave amplitude Bo is set as the representative value. It is assumed that the representative value of the second cuff 51 of the avascularization side after avascularization is the pulse wave amplitude Bc acquired at time t2. The representative values Ao and Bo of the non-avascularization side before and after avascularization are identical with each other. Therefore, the vascular endothelial function is evaluated by the comparison result Bc/Ac of the representative values of the avascularization side.

In the embodiment, the pulse wave amplitude is measured during the process of depressurizing the cuff pressure. Alternatively, the measurement may be performed during the process of pressurizing the cuff pressure.

Furthermore, the pulse wave amplitude for each beat may be measured in a state where the cuff pressure is kept constant (at a constant pressure). For example, the constant pressure may be about 20 mmHg.

The embodiment is configured so that avascularization is performed by the first cuff 11, and the pulse wave for each beat is measured by the second cuff 51. Alternatively, the first cuff 11 and the second cuff 51 may be configured as a single cuff. In the alternative, the configuration of Fig. 1 is modified so that the first cuff 11, the first pump 12, the first valve 13, and the first pressure sensor 14 are removed away, and the second cuff 51, the second pump 52, the second valve 53, and the second pressure sensor 54 are provided also with the function for avascularization. The third cuff 71, the third pump 72, the third valve 73, and the third pressure sensor 74 are not changed.

The operation of performing avascularization on the subject for a predetermined time as described above means the followings. Normal vascular endothelial cells are caused to produce vasodepressor materials such as nitric monoxide (NO) by stimulation due to a blood flow or medication. When the vascular endothelial function is lowered, the ability of producing the vasodepressor materials is lowered. With a measurement of the degree of vessel expansion due to avascularization, it is possible to evaluate the vascular endothelial function.

The relationships among the inner vascular pressure, the cuff pressure, and the pulse wave amplitude contained in the cuff pressure signal can be described as follows. First, it is known that, when the inner vascular pressure and the cuff pressure are equal to each other, the vascular compliance is maximum. When the cuff pressure is equal to the mean blood pressure, the difference between the maximal and minimal blood pressures in a vessel, i.e., the vessel volumetric change due to the pulse pressure is maximum. In the measurement periods Tp and Ta before and after avascularization, therefore, the maximal pulse wave amplitudes Ac and Bc are measured by using the second cuff 51, and set as representative pulse wave amplitudes of the avascularization side, and the maximal pulse wave amplitudes Ao and Bo are measured by using the third cuff 71, and set as representative pulse wave amplitudes of the non-avascularization side.

Alternatively, the mean blood pressure may be previously measured, and the pulse wave amplitude at the application of the cuff pressure corresponding to the mean blood pressure may be set as the representative value. In any case, the representative value is obtained by applying statistical processing on the pulse wave amplitudes in the measurement periods Tp and Ta in which the second cuff 51 and the third cuff 71 are used.

In the above description, the measurement of the pulse wave amplitude by using the second cuff 51 and the third cuff 71 is performed one time respectively before and after the pressurization period T for avascularization. Alternatively, a plurality of measurements may be performed. In the alternative, the numbers of measurements before and after avascularization may be different from each other.

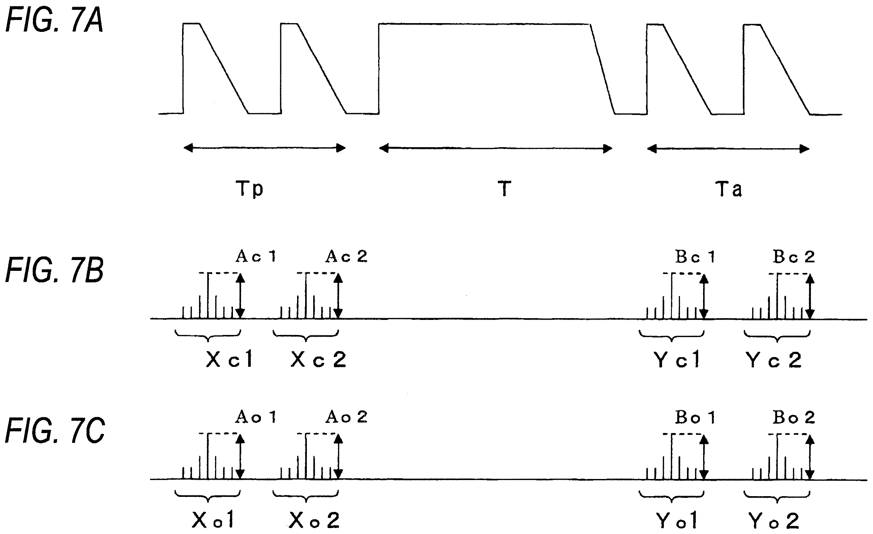

Figs. 7A to 7C show an example in which, in each of the measurement periods Tp and Ta, the pulse wave amplitude is measured two times. In this example, during the measurement period Tp shown in Fig. 7A, two pulse wave measurements are performed by using the second cuff 51, and the pulse wave detector 32 obtains measured-value groups Xc1 and Xc2. The analyzer 33 performs statistical processing on the measured-value groups Xc1 and Xc2 to obtain representative values. In this example, maximal pulse wave amplitudes Ac1 and Ac2 are obtained as representative values as shown in Fig. 7B.

Similarly, during the measurement period Tp, two measurements are performed by using the third cuff 71, and the pulse wave detector 32 obtains measured-value groups Xo1 and Xo2. The analyzer 33 performs statistical processing on the measured-value groups Xo1 and Xo2 to obtain representative values. In this example, maximal pulse wave amplitudes Ao1 and Ao2 are obtained as representative values as shown in Fig. 7C.

Also during the measurement period Ta shown in Fig. 7A, two measurements are performed by using the second cuff 51, and the pulse wave detector 32 obtains measured-value groups Yc1 and Yc2. The analyzer 33 performs statistical processing on the measured-value groups Yc1 and Yc2, and maximal pulse wave amplitudes Bc1 and Bc2 are obtained as representative values as shown in Fig. 7B.

Similarly, during the measurement period Ta shown in Fig. 7A, two measurements are performed by using the third cuff 71, and the pulse wave detector 32 obtains measured-value groups Yo1 and Yo2. The analyzer 33 performs statistical processing on the measured-value groups Yo1 and Yo2, and maximal pulse wave amplitudes Bo1 and Bo2 are obtained as representative values as shown in Fig. 7C.

The analyzer 33 performs statistical processing for obtaining the respective mean values on the maximum pulse wave amplitudes Ac1 and Ac2 and maximum pulse wave amplitudes Bc1 and Bc2, and the maximum pulse wave amplitudes Ao1 and Ao2 and maximum pulse wave amplitudes Bo1 and Bo2 which are obtained in the above-described processes, to obtain final representative values. By using the obtained representative values, the representative values of the avascularization side before and after avascularization are compared with each other, and a result of the comparison can be corrected by the representative values of the non-avascularization side before and after avascularization. As a result of the above-described measurements and processing, errors can be leveled, and accurate evaluation is enabled.

Other measurement technique and statistical processing will be described. The vessel expansion due to avascularization by the first cuff 11 and its release, i.e., the increase of the vessel diameter is gradually increased after the release of the avascularization to reach the peak after several tens of seconds, and thereafter is reduced for several minutes. Based on the peak, it is possible to evaluate the degree of vessel expansion. After avascularization using the first cuff 11, the maximum value of the pulse wave amplitude is measured several times by using the second cuff 51 and the third cuff 71, and a maximum one of the maximum values is set as the pulse wave amplitude in the vessel expansion, thereby obtaining the peak of the vessel expansion.

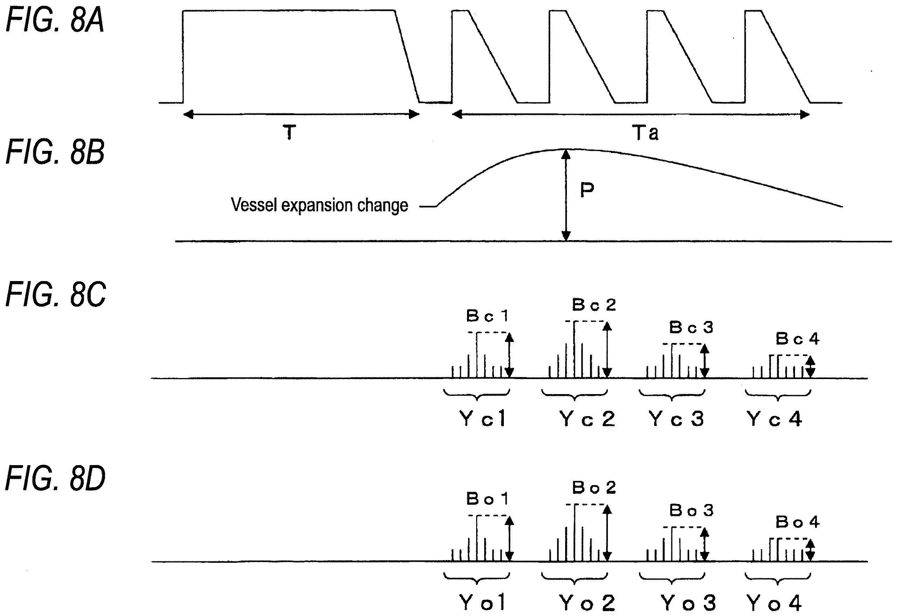

As shown in Fig. 8A, specifically, the measurement period Ta for measuring the pulse wave amplitude by using the second cuff 51 and the third cuff 71 is realized after the pressurization period T using the first cuff 11. In the example, in accordance with four measurements using the second cuff 51, measured-value groups Yc1, Yc2, Yc3, and Yc4 are obtained as shown in Fig. 8C. In this case, the vessel diameter changes as shown Fig. 8B, and it is estimated that a peak P exists in the vicinity of the measured-value group Yc2. The analyzer 33 performs statistical processing on the measured-value groups Yc1, Yc2, Yc3, and Yc4, and, as shown in Fig. 8C, obtains maximal pulse wave amplitudes Bc1, Bc2, Bc3, and Bc4 as candidates for the representative value.

The analyzer 33 compares the levels of the maximal pulse wave amplitudes Bc1, Bc2, Bc3, and Bc4 with one another, and selects the maximal pulse wave amplitude Bc2 as the representative value.

Similarly, in accordance with four measurements using the third cuff 71, measured-value groups Yo1, Yo2, Yo3, and Yo4 are obtained. The analyzer 33 performs statistical processing on the measured-value groups Yo1, Yo2, Yo3, and Yo4, and, as shown in Fig. 8D, obtains maximal pulse wave amplitudes Bo1, Bo2, Bo3, and Bo4 as candidates for the representative value.

The analyzer 33 compares the levels of the maximal pulse wave amplitudes Bo1, Bo2, Bo3, and Bo4 with one another, and selects the maximal pulse wave amplitude Bo2 as the representative value. The maximal pulse wave amplitude Bc2 which is maximum in the avascularization side is corrected by the maximal pulse wave amplitude Bo2 which is maximum in the non-avascularization side, and also the maximal pulse wave amplitude which is obtained by the measurement before the pressurization period T is corrected as described above. The analyzer 33 performs comparison by using the corrected results, and calculates a ratio of the correction results before and after the pressurization period T. The calculated result is displayed together with the obtained blood pressure value on the displaying unit 40. As described above, the ratio may be obtained in first, and then the comparison may be performed.

The analyzer 33 performs processing in which the vascular compliance is obtained, in addition to the processings in which a result of comparison of pulse waves, and blood pressures are obtained. The analyzer 33 divides the detected pulse wave amplitude by the difference (pulse pressure) between the systolic blood pressure and the diastolic blood pressure, to obtain the vascular compliance C. Although the phenomenon in which, when the inner vascular pressure and the cuff pressure are equal to each other, the vascular compliance is maximum is already known, the compliance which is to be obtained in this case is not restricted to a value obtained when the inner vascular pressure and the cuff pressure are equal to each other. During the measurement periods Tp and Ta for measuring the pulse wave amplitude which are set before and after the pressurization period T, compliances based on the amplitudes of corrected representative values which are respectively obtained in the periods may be obtained. The obtained compliances are displayed on the displaying unit 40, independently or together with the ratio of the pulse wave amplitudes which have been described, and obtained blood pressure values. In this case, data of the measured compliances may be caused to remain as history information, the information may be formed as a table or a graph, and the trend may be displayed on the displaying unit 40.

## Claims

1. An apparatus for evaluating a vascular endothelial function comprising:
a first cuff (11) adapted to be wrapped around an upper or lower limb on the right or left side of a subject;
a second cuff (51) adapted to be wrapped around the same limb, around which the first cuff (11) is to be wrapped;
a third cuff (71) adapted to be wrapped around the corresponding limb on the opposite side to the one limb around which the first cuff (11) and the second cuff (51) are to be wrapped;
a first pressure sensor (54) and second pressure sensor (74) connected to the second cuff (51) and third cuff (71) respectively, a cuff pressure controller (20) configured to control pressures that are applied to the first cuff (11), the second cuff (51) and the third cuff (71),
a cuff pressure detector (31) configured to detect, from an output of the first pressure sensor (54) connected to the second cuff (51), and an output of the second pressure sensor (74) connected to the third cuff (71), cuff pressures in the second cuff (51) and the third cuff (71);
a pulse wave detector (32) configured to detect respective pulse waves at the second cuff (51) and the third cuff (71) from outputs of the cuff pressure detector; and
an analyzer (33) configured to analyse the detected pulse waves to evaluate a vascular endothelial function,
wherein the cuff pressure controller (20) is configured to control the cuff pressure of the first cuff to avascularize the one limb around which the first cuff (11) is wrapped, for a predetermined time period,
the pulse wave detector (32) is configured to detect, before and after the avascularization a pulse wave corresponding to the second cuff (51), and a pulse wave corresponding to the third cuff (71), avascularization in a state where the cuff pressures of the second (51) and third cuffs (71) are controlled to respective predetermined pressures by the cuff pressure controller (20),
the analyzer (33) is configured to correct the pulse waves which correspond to the second cuff (51) and are detected before and after the avascularization, by the pulse waves which correspond to the third cuff (71) and are detected before and after the avascularization, and
the analyzer (33) is configured to evaluate the vascular endothelial function based on the corrected pulse waves corresponding to the second cuff (51).

2. An apparatus for evaluating a vascular endothelial function wherein the apparatus includes:
a second cuff (51) adapted to be wrapped around an upper or lower limb on the right or left side of a subject;
a third cuff (71) adapted to be wrapped around the corresponding limb on the opposite side to the one limb around which the second cuff (51) is to be wrapped;
a first pressure sensor (54) and a second pressure sensor (74) connected to the second cuff (51) and third cuff (71) respectively, a cuff pressure controller (20) configured to control pressures that are applied to the second cuff (51) and the third cuff (71),
a cuff pressure detector (31) configured to detect, from an output of the first pressure sensor (54) connected to the second cuff (51), and an output of the second pressure sensor (74) connected to the third cuff (71), cuff pressures in the second cuff (51) and the third cuff (71);
a pulse wave detector (32) configured to detect respective pulse waves at the second cuff (51) and the third cuff (71) from outputs of the cuff pressure detector and
an analyzer (33) configured to analyse the detected pulse waves to evaluate a vascular endothelial function,
wherein the cuff pressure controller (20) is configured to control the cuff pressure of the second cuff (51) to avascularize the one limb around which the second cuff (51) is wrapped, for a predetermined time period,
the pulse wave detector (32) is configured to detect, before and after avascularization, a pulse wave corresponding to the second cuff (51), and a pulse wave corresponding to the third cuff (71), in a state where the cuff pressures of the second (51) and third cuffs (71) are controlled to respective predetermined pressures by the cuff pressure controller (20),
the analyzer (33) is configured to correct the pulse waves which correspond to the second cuff (51) and are detected before and after the avascularization, by the pulse waves corresponding to the third cuff (71) and detected before and after the avascularization, and
the analyzer (33) is configured to evaluate the vascular endothelial function based on the corrected pulse waves corresponding to the second cuff (51).

3. The apparatus for evaluating a vascular endothelial function according to claim 1 or 2, wherein the pulse waves handled in the analyzer (33) are pulse wave indexes, and the pulse wave indexes are a parameter indicating a feature of a pulse wave.

4. The apparatus for evaluating a vascular endothelial function according to claim 3, wherein the analyzer is configured to correct a result of a comparison between the pulse wave indexes corresponding to the second cuff (51) and detected before and after the avascularization.

5. The apparatus for evaluating a vascular endothelial function according to claim 4, wherein the comparison result is ratios of the pulse wave indexes of the second cuff (51) and the third cuff (71) before and after the avascularization, and the correction corrects the ratio of the pulse wave indexes corresponding to the second cuff (51) by using the ratio of the pulse wave indexes corresponding to the third cuff (71).

6. The apparatus for evaluating a vascular endothelial function according to claim 4 or 5, wherein the comparison and the correction are performed by using values of the pulse wave indexes corresponding to the second cuff (51) or the third cuff (71), being obtained at a required timing, and values of the pulse wave indexes corresponding to the third cuff (71) or the second cuff (51), being obtained at the required timing.

7. The apparatus for evaluating a vascular endothelial function according to claim 4 or 5, wherein the comparison and the correction are performed by using values of the pulse wave indexes corresponding to the third cuff (71), and values of the pulse wave indexes corresponding to the second cuff (51) and equal to the values of the pulse wave indexes corresponding to the third cuff (71).

8. The apparatus for evaluating a vascular endothelial function according to any one of claims 3 to 7, wherein the pulse wave indexes are a maximum value of a pulse wave amplitude, or a mean value in the vicinity of a maximum value.

9. The apparatus for evaluating a vascular endothelial function according to any one of claims 1 to 8, wherein the analyzer (33) further performs a process of obtaining a systolic blood pressure and a diastolic blood pressure, and dividing an amplitude of the corrected pulse wave by a difference between the systolic blood pressure and the diastolic blood pressure, thereby obtaining a compliance of a blood vessel.

## Patentansprüche

1. Gerät zum Bewerten einer vaskulären Endothelfunktion, umfassend:
eine erste Manschette (11), die dazu ausgelegt ist, um eine obere oder untere Gliedmaße an der rechten oder linken Seite eines Subjekts gewickelt zu sein;
eine zweite Manschette (51), die dazu ausgelegt ist, um die gleiche Gliedmaße gewickelt zu sein, um die die erste Manschette (11) gewickelt ist;
eine dritte Manschette (71), die dazu ausgelegt ist, um die entsprechende Gliedmaße an der entgegengesetzten Seite zu der Gliedmaße gewickelt zu sein, um die die erste Manschette (11) und die zweite Manschette (51) zu wickeln sind;
einen ersten Drucksensor (54) und einen zweiten Drucksensor (74), die mit der zweiten Manschette (51) beziehungsweise der dritten Manschette (71) verbunden sind, und ein Manschettendruck-Steuergerät (20), das dazu ausgelegt ist, Drücke zu steuern, die an die erste Manschette (11), die zweite Manschette (51) und die dritte Manschette (71) angelegt werden,
einen Manschettendruckdetektor (31), der dazu ausgelegt ist, anhand einer Ausgabe des mit der zweiten Manschette (51) verbundenen ersten Drucksensors (54) und einer Ausgabe des mit der dritten Manschette (71) verbundenen zweiten Drucksensors (74) Manschettendrücke in der zweiten Manschette (51) und der dritten Manschette (71) zu erfassen;
einen Pulswellendetektor (32), der dazu ausgelegt ist, jeweilige Pulswellen an der zweiten Manschette (51) und der dritten Manschette (71) aus Ausgaben des Manschettendruckdetektors zu erfassen; und
einen Analysator (33), der dazu ausgelegt ist, die erfassten Pulswellen zu analysieren, um eine vaskuläre Endothelfunktion zu bewerten,
wobei das Manschettendruck-Steuergerät (20) dazu ausgelegt ist, den Manschettendruck der ersten Manschette zu steuern, um die eine Gliedmaße, um die die erste Manschette (11) gewickelt ist, für eine vorbestimmte Zeitdauer zu avaskularisieren,
wobei der Pulswellendetektor (32) dazu ausgelegt ist, vor und nach der Avaskularisierung eine Pulswelle entsprechend der zweiten Manschette (51) und eine Pulswelle entsprechend der dritten Manschette (71) in einem Zustand zu erfassen, in dem die Manschettendrücke der zweiten (51) und der dritten (71) Manschette durch das Manschettendruck-Steuergerät (20) auf jeweilige vorbestimmte Drücke gesteuert sind,
wobei der Analysator (33) dazu ausgelegt ist, die Pulswellen, die der zweiten Manschette (51) entsprechen und vor und nach der Avaskularisierung erfasst sind, durch die Pulswellen zu korrigieren, die der dritten Manschette (71) entsprechen und vor und nach der Avaskularisierung erfasst sind, und
wobei der Analysator (33) dazu ausgelegt ist, die vaskuläre Endothelfunktion basierend auf den korrigierten Pulswellen entsprechend der zweiten Manschette (51) zu bewerten.

2. Gerät zum Bewerten einer vaskulären Endothelfunktion, wobei das Gerät umfasst:
eine zweite Manschette (51), die dazu ausgelegt ist, um eine obere oder untere Gliedmaße an der rechten oder linken Seite eines Subjekts gewickelt zu sein;
eine dritte Manschette (71), die dazu ausgelegt ist, um die entsprechende Gliedmaße an der entgegengesetzten Seite zu der einen Gliedmaße gewickelt zu sein, um die die zweite Manschette (51) zu wickeln ist;
einen ersten Drucksensor (54) und einen zweiten Drucksensor (74), die mit der zweiten Manschette (51) beziehungsweise der dritten Manschette (71) verbunden sind, und ein Manschettendruck-Steuergerät (20), das dazu ausgelegt ist, Drücke zu steuern, die an die zweite Manschette (51) und die dritte Manschette (71) angelegt sind,
einen Manschettendruckdetektor (31), der dazu ausgelegt ist, anhand einer Ausgabe des mit der zweiten Manschette (51) verbundenen ersten Drucksensors (54) und einer Ausgabe des mit der dritten Manschette (71) verbundenen zweiten Drucksensors (74) Manschettendrücke in der zweiten Manschette (51) und der dritten Manschette (71) zu erfassen;
einen Pulswellendetektor (32), der dazu ausgelegt ist, jeweilige Pulswellen an der zweiten Manschette (51) und der dritten Manschette (71) anhand von Ausgaben des Manschettendruckdetektors zu erfassen, und
einen Analysator (33), der dazu ausgelegt ist, die erfassten Pulswellen zu analysieren, um eine vaskuläre Endothelfunktion zu bewerten,
wobei das Manschettendruck-Steuergerät (20) dazu ausgelegt ist, den Manschettendruck der zweiten Manschette (51) zu steuern, um die eine Gliedmaße, um die die zweite Manschette (51) gewickelt ist, für eine vorbestimmte Zeitdauer zu avaskularisieren,
wobei der Pulswellendetektor (32) dazu ausgelegt ist, vor und nach der Avaskularisierung eine Pulswelle entsprechend der zweiten Manschette (51) und eine Pulswelle entsprechend der dritten Manschette (71) in einem Zustand zu erfassen, in dem die Manschettendrücke der zweiten (51) und der dritten (71) Manschette durch das Manschettendruck-Steuergerät (20) auf jeweilige vorbestimmte Drücke gesteuert sind,
wobei der Analysator (33) dazu ausgelegt ist, die Pulswellen, die der zweiten Manschette (51) entsprechen und vor und nach der Avaskularisierung erfasst sind, durch die Pulswellen zu korrigieren, die der dritten Manschette (71) entsprechen und vor und nach der Avaskularisierung erfasst sind, und
wobei der Analysator (33) dazu ausgelegt ist, die vaskuläre Endothelfunktion basierend auf den korrigierten Pulswellen entsprechend der zweiten Manschette (51) zu bewerten.

3. Gerät zum Bewerten einer vaskulären Endothelfunktion nach Anspruch 1 oder 2, wobei die in dem Analysator (33) verarbeiteten Pulswellen Pulswellenindizes sind, und die Pulswellenindizes ein Parameter sind, der ein Merkmal einer Pulswelle angibt.

4. Gerät zum Bewerten einer vaskulären Endothelfunktion nach Anspruch 3, wobei der Analysator dazu ausgelegt ist, ein Ergebnis eines Vergleichs zwischen den Pulswellenindizes zu korrigieren, die der zweiten Manschette (51) entsprechen und vor und nach der Avaskularisierung erfasst sind.

5. Gerät zum Bewerten einer vaskulären Endothelfunktion nach Anspruch 4, wobei das Vergleichsergebnis Verhältnisse der Pulswellenindizes der zweiten Manschette (51) und der dritten Manschette (71) vor und nach der Avaskularisierung ist, und die Korrektur das Verhältnis der Pulswellenindizes entsprechend der zweiten Manschette (51) unter Verwendung des Verhältnisses der Pulswellenindizes entsprechend der dritten Manschette (71) korrigiert.

6. Gerät zum Bewerten einer vaskulären Endothelfunktion nach Anspruch 4 oder 5, wobei der Vergleich und die Korrektur unter Verwendung von Werten der Pulswellenindizes durchgeführt werden, die der zweiten Manschette (51) oder der dritten Manschette (71) entsprechen und an einem benötigten Zeitpunkt erhalten sind, und von Werten der Pulswellenindizes, die der dritten Manschette (71) oder der zweiten Manschette (51) entsprechen und an dem benötigten Zeitpunkt erhalten sind.

7. Gerät zum Bewerten einer vaskulären Endothelfunktion nach Anspruch 4 oder 5, wobei der Vergleich und die Korrektur unter Verwendung von Werten der Pulswellenindizes durchgeführt werden, die der dritten Manschette (71) entsprechen, und von Werten der Pulswellenindizes, die der zweiten Manschette (51) entsprechen und gleich den Werten der Pulswellenindizes sind, die der dritten Manschette (71) entsprechen.

8. Gerät zum Bewerten einer vaskulären Endothelfunktion nach einem der Ansprüche 3 bis 7, wobei die Pulswellenindizes ein Maximalwert einer Pulswellenamplitude, oder ein Mittelwert in der Nähe eines Maximalwerts sind.

9. Gerät zum Bewerten einer vaskulären Endothelfunktion nach einem der Ansprüche 1 bis 8, wobei der Analysator (33) ferner eine Verarbeitung zum Erhalten eines systolischen Blutdrucks und eines diastolischen Blutdrucks durchführt, und zum Dividieren einer Amplitude der korrigierten Pulswelle durch eine Differenz zwischen dem systolischen Blutdruck und dem diastolischen Blutdruck, wodurch eine Nachgiebigkeit eines Blutgefäßes erhalten wird.

## Revendications

1. Appareil d'évaluation d'une fonction endothéliale vasculaire qui comprend :
un premier brassard (11) adapté pour être placé autour d'un membre supérieur ou inférieur sur le côté droit ou gauche d'un sujet ;
un second brassard (51) adapté pour être placé autour dudit membre autour duquel le premier brassard (11) doit être placé ;
un troisième brassard (71) adapté pour être placé autour du membre correspondant sur le côté opposé audit membre autour duquel le premier brassard (11) et le second brassard (51) doivent être placés ;
un premier capteur de pression (54) et un second capteur de pression (74) reliés au second brassard (51) et au troisième brassard (71), respectivement, un contrôleur de pression de brassard (20) étant configuré pour contrôler les pressions qui sont appliquées au premier brassard (11), au second brassard (51) et au troisième brassard (71),
un détecteur de pression de brassard (31) configuré pour détecter, à partir d'une sortie du premier capteur de pression (54) relié au second brassard (51), et d'une sortie du second capteur de pression (74) relié au troisième brassard (71), les pressions de brassard dans le second brassard (51) et le troisième brassard (71) ;
un détecteur d'onde d'impulsion (32) configuré pour détecter des ondes d'impulsions respectives au niveau du second brassard (51) et du troisième brassard (71) à partir des sorties du détecteur de pression de brassard ;
et
un analyseur (33) configuré pour analyser les ondes d'impulsions détectées afin d'évaluer une fonction endothéliale vasculaire,
dans lequel le contrôleur de pression de brassard (20) est configuré pour contrôler la pression de brassard du premier brassard afin d'avasculariser le membre autour duquel le premier brassard (11) est placé, pendant une durée prédéterminée,
le détecteur d'onde d'impulsion (32) est configuré pour détecter, avant et après l'avascularisation, une onde d'impulsion qui correspond au second brassard (51), et une onde d'impulsion qui correspond au troisième brassard (71), dans un état dans lequel les pressions de brassard du second (51) et du troisième brassards (71) sont contrôlées selon des pressions prédéterminées respectives par le contrôleur de pression de brassard (20),
l'analyseur (33) est configuré pour corriger les ondes d'impulsions qui correspondent au second brassard (51) et sont détectées avant et après l'avascularisation, en fonction des ondes d'impulsions qui correspondent au troisième brassard (71) et sont détectées avant et après l'avascularisation, et
l'analyseur (33) est configuré pour évaluer la fonction endothéliale vasculaire sur la base des ondes d'impulsions corrigés qui correspondent au second brassard (51).

2. Appareil d'évaluation d'une fonction endothéliale vasculaire, qui comprend :
un second brassard (51) adapté pour être placé autour d'un membre supérieur ou inférieur sur le côté droit ou gauche d'un sujet ;
un troisième brassard (71) adapté pour être placé autour du membre correspondant sur le côté opposé au membre autour duquel le second brassard (51) doit être placé ;
un premier capteur de pression (54) et un second capteur de pression (74) reliés au second brassard (51) et au troisième brassard (71), respectivement, un contrôleur de pression de brassard (20) configuré pour contrôler les pressions qui sont appliquées au second brassard (51) et au troisième brassard (71), un détecteur de pression de brassard (31) configuré pour détecter, à partir d'une sortie du premier capteur de pression (54) relié au second brassard (51), et d'une sortie du second capteur de pression (74) relié au troisième brassard (71), les pressions de brassard dans le second brassard (51) et le troisième brassard (71) ;
un détecteur d'onde d'impulsion (32) configuré pour détecter des ondes d'impulsions respectives au niveau du second brassard (51) et du troisième brassard (71) à partir des sorties du détecteur de pression de brassard ;
et
un analyseur (33) configuré pour analyser les ondes d'impulsions détectées afin d'évaluer une fonction endothéliale vasculaire,
dans lequel le contrôleur de pression de brassard (20) est configuré pour contrôler la pression de brassard du second brassard (51) afin d'avasculariser le membre autour duquel le second brassard (51) est placé, pendant une durée prédéterminée,
le détecteur d'onde d'impulsion (32) est configuré pour détecter, avant et après l'avascularisation, une onde d'impulsion qui correspond au second brassard (51), et une onde d'impulsion qui correspond au troisième brassard (71), dans un état dans lequel les pressions de brassard du second (51) et du troisième brassards (71) sont contrôlées selon des pressions prédéterminées respectives par le contrôleur de pression de brassard (20),
l'analyseur (33) est configuré pour corriger les ondes d'impulsions qui correspondent au second brassard (51) et sont détectées avant et après l'avascularisation, en fonction des ondes d'impulsions qui correspondent au troisième brassard (71) et sont détectées avant et après l'avascularisation, et
l'analyseur (33) est configuré pour évaluer la fonction endothéliale vasculaire sur la base des ondes d'impulsions corrigés qui correspondent au second brassard (51).

3. Appareil d'évaluation d'une fonction endothéliale vasculaire selon la revendication 1 ou 2, dans lequel les ondes d'impulsions gérées dans l'analyseur (33) sont des indices d'ondes d'impulsions, et les indices d'ondes d'impulsions sont un paramètre qui indique une caractéristique d'une onde d'impulsion.

4. Appareil d'évaluation d'une fonction endothéliale vasculaire selon la revendication 3, dans lequel l'analyseur est configuré pour corriger un résultat de comparaison entre les indices d'ondes d'impulsions qui correspondent au second brassard (51) et sont détectés avant et après l'avascularisation.

5. Appareil d'évaluation d'une fonction endothéliale vasculaire selon la revendication 4, dans lequel le résultat de comparaison est composé de rapports entre les indices d'ondes d'impulsions du second brassard (51) et du troisième brassard (71) avant et après l'avascularisation, et la correction corrige le rapport entre les indices d'ondes d'impulsions qui correspondent au second brassard (51) en utilisant le rapport entre les indices d'ondes d'impulsions qui correspondent au troisième brassard (71).

6. Appareil d'évaluation d'une fonction endothéliale vasculaire selon la revendication 4 ou 5, dans lequel la comparaison et la correction sont effectuées à l'aide des valeurs des indices d'ondes d'impulsions qui correspondent au second brassard (51) ou au troisième brassard (71), qui sont obtenues à un moment requis, et des valeurs des indices d'ondes d'impulsions qui correspondent au troisième brassard (71) ou au second brassard (51), qui sont obtenues au moment requis.

7. Appareil d'évaluation d'une fonction endothéliale vasculaire selon la revendication 4 ou 5, dans lequel la comparaison et la correction sont effectuées à l'aide des valeurs des indices d'ondes d'impulsions qui correspondent au troisième brassard (71), et des valeurs des indices d'ondes d'impulsions qui correspondent au second brassard (51) et sont égales aux valeurs des indices d'ondes d'impulsions qui correspondent au troisième brassard (71).

8. Appareil d'évaluation d'une fonction endothéliale vasculaire selon l'une quelconque des revendications 3 à 7, dans lequel les indices d'ondes d'impulsions sont une valeur maximale d'une amplitude d'onde d'impulsion,, ou une valeur moyenne proche d'une valeur maximale.

9. Appareil d'évaluation d'une fonction endothéliale vasculaire selon l'une quelconque des revendications 1 à 8, dans lequel l'analyseur (33) effectue en outre un processus d'obtention d'une pression artérielle systolique et d'une pression artérielle diastolique, et de division d'une amplitude de l'onde d'impulsion corrigée par une différence entre la pression artérielle systolique et la pression artérielle diastolique, afin d'obtenir une conformité du vaisseau sanguin.
